(19) Europäisches Patentamt
European Patent Office
Office européen des brevets

(11) Veröffentlichungsnummer: **0 324 983**
**B1**

(12) # EUROPÄISCHE PATENTSCHRIFT

(45) Veröffentlichungstag der Patentschrift: **14.11.90**

(51) Int. Cl.[5]: **B01J 23/46**, B01J 23/64, C07C 209/68

(21) Anmeldenummer: **88121824.2**

(22) Anmeldetag: **29.12.88**

(54) **Ruthenium-Trägerkatalysator, seine Herstellung und sein Einsatz bei der Herstellung von gegebenenfalls substituiertem Cyclohexylamin und gegebenenfalls substituiertem Dicyclohexylamin.**

(30) Priorität: **22.01.88 DE 3801755**

(43) Veröffentlichungstag der Anmeldung:
**26.07.89 Patentblatt 89/30**

(45) Bekanntmachung des Hinweises auf die Patenterteilung:
**14.11.90 Patentblatt 90/46**

(84) Benannte Vertragsstaaten:
**BE CH DE FR GB IT LI**

(56) Entgegenhaltungen:
**EP-A- 0 001 425**
**EP-A- 0 053 818**
**EP-A- 0 053 819**
**FR-A- 2 232 359**
**FR-A- 2 234 922**
**FR-A- 2 298 366**

(73) Patentinhaber: **BAYER AG,**
**D-5090 Leverkusen 1 Bayerwerk(DE)**

(72) Erfinder: **Immel, Otto, Dr., Immenhofweg 26,**
**D-4150 Krefeld(DE)**
Erfinder: **Schwarz, Hans-Helmut, Dr., Ratherstrasse 90,**
**D-4150 Krefeld 1(DE)**
Erfinder: **Thiel, Reinhard, Dr., Buschstrasse 215a,**
**D-4150 Krefeld 1(DE)**

EP 0 324 983 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die Erfindung betrifft einen Ruthenium-Katalysator auf einem Träger, ein Verfahren zu seiner Herstellung und ein Verfahren zur Herstellung von gegebenenfalls substituiertem Cyclohexylamin und gegebenenfalls substituiertem Dicyclohexylamin durch katalytische Hydrierung von gegebenenfalls substituiertem Anilin unter Einsatz eines solchen Katalysators.

Es ist bekannt, Cyclohexylamin durch Druckhydrierung von Anilin herzustellen. Für diese Hydrierung werden Kobaltkatalysatoren, die einen basischen Zusatz enthalten (GB 969 542), sowie Raney-Kobalt (JP 68/03180) eingesetzt. Nach US 3.636.108 wird zur Kernhydrierung aromatischer Aminoverbindungen ein mit Alkali moderierter Ruthenium-Katalysator auf einem inerten Trägermaterial verwendet, wobei man zusätzlich $NH_3$ und gegebenenfalls ein Lösungsmittel einsetzt. Ein weiteres Verfahren zur Druckhydrierung von Anilin zu Cyclohexylamin ist in DE-AS 11 06 319 beschrieben, bei dem ebenfalls ein Ruthenium-Katalysator verwendet wird. In diesem Verfahren wird mitentstehendes Dicyclohexylamin dem Einsatzmaterial wieder zugesetzt; das Verfahren bringt beträchtliche Verluste durch die gleichzeitige Entstehung von Cyclohexan. Im Gegensatz zu den bisher genannten Veröffentlichungen hält EP 53 818 Palladium-Trägerkatalysatoren zur Druckhydrierung von Anilin für günstiger als Ruthenium-Katalysatoren.

Bei den bekannten Druckhydrierungsverfahren von Anilin entsteht das Dicyclohexylamin neben dem Cyclohexylamin lediglich als Nebenprodukt. Um Dicyclohexylamin in größeren Mengen zu erhalten, wird es nach separaten Verfahren hergestellt. So kann es beispielsweise durch Druckhydrierung von Diphenylamin unter Verwendung eines Ruthenium-$Al_2O_3$-Katalysators gewonnen werden (DE-AS 11 06 319). Weiterhin entsteht Dicyclohexylamin bei der Umsetzung von Cyclohexanon mit Cyclohexylamin in Gegenwart von Palladium auf Kohle bei einem Wasserstoffdruck von 4 bar (FR 1.333.692). In einem umständlichen Verfahren kann Dicyclohexylamin aus dem Hydrierprodukt des Anilins an einem Nickelkatalysator durch fraktioniertes Auskondensieren gewonnen werden. Aus dem zurückbleibenden Gemisch wird ein Teil des mitentstandenen Ammoniaks entfernt und der Rest wieder in die Reaktion zurückgeführt (DE-PS 805 518).

Ein gemeinsames Problem aller Verfahren zur Kernhydrierung aromatischer Amine besteht in der zum Teil beträchtlichen Bildung von Cyclohexan als nicht weiter verwendbares Abfallprodukt.

Es bestand daher der Wunsch, ein neues, auch in technischem Maßstab brauchbares Verfahren zu entwickeln, nach welchem in einer Reaktionsstufe sowohl Cyclohexylamin als auch Dicyclohexylamin in einem gewünschten Mengenverhältnis hergestellt werden kann, in welchem der Verlust durch die Bildung von Cyclohexan zurückgedrängt wird und in welchem weiterhin die Standzeit des verwendeten Katalysators verbessert ist.

Überraschenderweise wurde gefunden, daß die genannten Anforderungen durch den Einsatz des im folgenden gekennzeichneten Ruthenium-Trägerkatalysator erfüllt werden, der insbesondere durch die Edelmetall-Kombination Ru/Pd, Ru/Pt oder Ru/Pd/Pt gekennzeichnet ist.

Die Erfindung betrifft demnach Ruthenium-Katalysatoren, die zusätzlich zum Ruthenium noch Palladium, Platin oder Palladium und Platin enthalten, auf einem mit Chrom und Mangan behandelten Träger aus der Gruppe von $Al_2O_3$ und Aluminiumspinell, der die Edelmetalle in einer Gesamtmenge von 0,05-5 Gew.-% und einem Gewichtsverhältnis Ru : Pd, Ru : Pt beziehungsweise Ru : Pd/Pt von 1:9 bis 9:1 enthält, wobei die Prozentsätze auf das Gesamtgewicht des Katalysators bezogen sind.

Die Gesamtmenge der Edelmetalle beträgt, bezogen auf das Gesamtgewicht des Katalysators, 0,05-5 Gew.-%, bevorzugt 0,05-3 Gew.-%, besonders bevorzugt 0,1-2 Gew.-%. Das Gewichtsverhältnis Ru : Pd, Ru : Pt beziehungsweise Ru : Pd/Pt reicht von 1:9-9:1, bevorzugt 2:8-8:2, besonders bevorzugt 3:7 bis 7:3.

Bis zu 20 % der Gesamtmenge an Ruthenium und Palladium bzw. Platin kann weiterhin durch andere Edelmetalle aus der Gruppe Iridium, Rhodium, Silber und Gold ersetzt sein.

Der erfindungsgemäße Katalysator kann weiterhin, wiederum bezogen auf das Gesamtgewicht des Katalysators, 0,01-10 Gew.-%, bevorzugt 0,1-5 Gew.-% einer basischen Alkalimetallverbindung enthalten. Solche basischen Alkalimetallverbindungen sind beispielsweise die Oxide, Hydroxide, Alkoholate oder Salze schwacher Säuren von Lithium, Natrium, Kalium, Rubidium oder Cäsium, bevorzugt die Hydroxide, Alkoholate oder Salze schwacher Säuren von Lithium, Natrium oder Kalium, besonders bevorzugt von Natrium oder Kalium. Schwache Säuren sind beispielsweise Kohlensäure, Essigsäure, Ameisensäure und andere Säuren, deren Alkalisalze alkalisch reagieren und sind weiterhin solche, die frei sind von Stickstoff, Halogen, Schwefel und anderen als Gifte für Hydrierkatalysatoren geltenden Elementen. Alkoholate sind beispielswiese solche des Methanols, Ethanols, Propanols, Butanols und anderer Alkohole. Der Zusatz einer basischen Alkalimetallverbindung zum erfindungsgemäßen Katalysator ist jedoch nicht zwingend nötig.

Der erfindungsgemäße Katalysator enthält als Träger Aluminiumoxid oder einen Aluminiumspinell, der mit Chrom und Mangan behandelt worden ist. Als Aluminiumoxid kommen insbesondere die $\alpha$- und die $\gamma$-Modifikationen in Frage. Aluminiumspinelle sind Verbindung der Formel $Me(II)Al_2O_4$, in denen Me(II) ein zweiwertiges Metallkation des Eisens, Zinks, Nickels, Kupfers, Kobalts, Cadmiums, Magnesiums oder anderer, bevorzugt des Magnesiums sein kann; ebenso kommt $LiAlO_2$ (Lithium-Aluminium-Spinell) in Frage. Das Aluminium kann teilweise durch dreiwertiges Eisen, Chrom oder Mangan ersetzt sein. Ein solcher Träger weist einen Gehalt an Chrom und Mangan von zusammen etwa 0,05-8 Gew.-%, bevorzugt 0,2-5 Gew.-%, bezogen auf das Gesamtgewicht des Katalysators auf. Das Gewichtsverhältnis von Chrom und Mangan beträgt etwa 5:1 bis 1:5, bevorzugt 10:9 bis 1:2. Solche mit Chrom und Mangan behandelten Träger sind aus EP 208 933 bekannt.

Zur Herstellung der beschriebenen erfindungsgemäßen Katalysatoren kann so vorgegangen werden, daß auf ein $Al_2O_3$ oder einen Aluminiumspinell in Form von Strangpreßlingen, Pillen oder Kugeln mit Abmessungen von etwa 2 - 10 mm Verbindungen des Chroms und Mangans aufgebracht werden, der so beaufschlagte Träger auf höhere Temperatur erhitzt wird, anschließend getrennt die Edelmetalle und gegebenenfalls die alkalischen Alkalimetallverbindungen aufgetragen werden und nach jedem Auftrag bei 90 - 130°C, gegebenenfalls im Vakuum, getrocknet wird.

Das Aufbringen des Chroms und Mangans auf den Katalysatorträger kann beispielsweise durch gemeinsames Ausfällen eines Mangan-Chrom-Hydroxidgemisches aus einer Chrom- und Mangansalz-Lösung mit Alkalilauge oder Ammoniak und gegebenenfalls anschließendes Auswaschen der löslichen Anteile mit Wasser erfolgen. Als Chrom-und Mangansalze kommen insbesondere die Sulfate, Chloride, Acetate und/oder Nitrate der genannten Elemente in Betracht. Die Abscheidung des Chroms und Mangans auf den Katalysatorträger kann auch als Ammonium-Mangan-Chromat oder Ammonium-Alkali-Mangan-Chromat aus einer Lösung von Mangan(II)-Salzen und Ammoniumbichromat mittels Ammoniak und/ode basischer Alkaliverbindungen erfolgen. Besonders gleichmäßige und fest haftende Abscheidungen erhält man, wenn die Basenzugabe langsam und gleichmäßig unter Vermeidung größerer Konzentrationsunterschiede erfolgt. Hierzu kann beispielsweise die Fällung mittels Harnstoff unter hydrolysierenden Bedingungen vorgenommen werden, wodurch die Bedingungen der langsamen Basenzugabe besonders gut gewährleistet sind. Der so hergestellte Chrom-Mangan-Niederschlag befindet sich als Schale auf dem Trägerkern aus $Al_2O_3$ oder Al-Spinell.

Nach dem Aufbringen der Chrom- und Manganverbindungen und der beschriebenen Ausfällung wird der so beaufschlagte Katalysatorträger im allgemeinen von löslichen Verbindungen freigewaschen, bevor er auf höhere Temperaturen (etwa 200-450°C, bevorzugt 250-350°C) erhitzt wird. Dieses Erhitzen erfolgt in einer Zeit von 1 - 120 Stunden. Während dieser Zeit kann die Temperatur im angegebenen Bereich erhöht werden. Nach dieser Temperung wird der mit Chrom und Mangan beaufschlagte Katalysatorträger mit den Edelmetallen und gegebenenfalls mit den alkalischen Alkalimetallverbindungen getränkt. Hierbei kann so vorgegangen werden, daß zunächst die Edelmetal le, beispielsweise in Form wäßriger Lösungen ihrer Chloride, Nitrate, Acetate oder anderer geeigneter Salze auf den Träger aufgetränkt werden, wobei nach einer Trocknung die Behandlung mit der Lösung einer basischen Alkalimetallverbindung erfolgt. Bei dieser Behandlung werden die Edelmetalle in Form ihrer Oxide oder Hydroxide ausgefällt. Nach einer abschließenden Trocknung steht der erfindungsgemäße Katalysator zur Verfügung. Er wird in bevorzugter Weise im Reaktor vor seinem Einsatz durch Behandlung mit Wasserstoff bei erhöhter Temperatur von 150 - 350°C aktiviert. Nach der Aktivierung kann es wünschenswert sein, Anionen, wie Chlorid, Nitrat, Acetat oder andere durch eine Wasserwäsche zu entfernen. Hierauf kann sich nochmals eine Behandlung mit basischen Alkalimetallverbindungen anschließen.

Man kann jedoch auch den mit Chrom und Mangan beaufschlagten Katalysatorträger zunächst mit der basischen Alkalimetallverbindung tränken, anschließend trocknen und auf den so vorbehandelten basisch eingestellten Katalysatorträger die genannten Salze der Edelmetalle auftragen, wobei im Moment der Tränkung auch die Ausfällung der Edelmetalle in Form ihrer Oxide oder Hydroxide erfolgt. Auch hierbei ist nach einer abschließenden Trocknung der Katalysator einsatzbereit und kann in der beschriebenen Weise bevorzugt vorab mit Wasserstoff bei erhöhter Temperatur aktiviert werden.

Je nachdem, ob der so hergestellte Katalysator die basischen Alkalimetallverbindungen enthalten soll oder nicht, kann eine anschließende Wasserwäsche entfallen oder durchgeführt werden.

Anstatt den genannten Träger zur Beaufschlagung mit den genannten Stoffen zu tränken, kann er auch mit geeigneten Lösungen besprüht werden. Die hierzu erforderlichen Arbeitsgeräte und die Einstellung der gewünschten Beaufschlagung durch Wahl der Menge und Konzentration der Lösungen der genannten Elemente sind dem Fachmann im Prinzip bekannt.

Der erfindungsgemäße Katalysator kann in hervorragender Weise zur Kernhydrierung von Anilin unter erhöhtem Druck eingesetzt werden. In besonders überraschender Weise läßt sich unter Verwendung des erfindungsgemäßen Katalysators in Abhängigkeit von der Hydriertemperatur die Menge des dabei mitentstehenden Dicyclohexylamins gegenüber dem Monocyclohexylamin verändern, wodurch eine gezielte Herstellung von Dicyclohexylamin in größeren Mengen möglich wird. Der erfindungsgemäße Katalysator zeigt gegenüber einem nicht mit Palladium und/oder Platin hergestellten reinen Ruthenium-Katalysator die für kontinuierliche technische Prozesse erforderliche hohe Standzeit.

Damit est erfindungsgemäß ein Verfahren zur Herstellung eines Gemisches von gegebenenfalls substituiertem Cyclohexylamin und gegebenenfalls substituiertem Dicyclohexylamin durch Hydrierung von gegebenenfalls substituiertem Anilin mit Wasserstoff in Gegenwart des oben be schriebenen Katalysators möglich, bei welchem man im Bereich von 80 bis 240°C, bevorzugt 100 bis 200°C bei einem Druck von 50 bis 500 bar, bevorzugt 100 bis 400 bar, besonders bevorzugt 150 bis 350 bar arbeitet.

Erfindungsgemäß kann die Menge des gewünschten Dicyclohexylamins durch Variation der Temperatur erreicht werden, wobei höhere Hydriertemperaturen einem höheren Anteil an Dicyclohexylamin entsprechen und umgekehrt. So erhält man beispielsweise bei einer Reaktionstemperatur von etwa 100°C

nur bis etwa 4 Gew.-% Dicyclohexylamin im Gemisch der kernhydrierten Amine, während bei einer Hydriertemperatur von etwa 200°C bis zu einer Menge von mehr als 50 % der hydrierten Amine in Form des Dicyclohexylamins vorliegen können.

Die Hydrierung am erfindungsgemäßen Katalysator kann diskontinuierlich oder kontinuierlich, in bevorzugter Weise kontinuierlich vorgenommen werden; hierbei arbeitet man mit einer fest angeordneten Katalysatorschüttung in der Rieselphase. Als Katalysatorbelastung wird eine Menge von 0,05 bis 2, bevorzugt 0,1 bis 0,5 kg Anilin pro Liter Katalysator pro Stunde eingestellt. Eine geringe Veränderung des erzielten Anteils an Dicyclohexylamin durch veränderte Aktivität des Katalysators im Laufe längerer Reaktionsperioden kann durch ein geringes Nachstellen der Reaktionstemperatur oder der anderen Parameter ausgeglichen werden. Diese Verhältnisse können anhand der Analytik des Reaktionsgemisches verfolgt werden.

Als Einsatzmaterialien kommen im Sinne der folgenden Reaktionsgleichung Anilin und substituierte Aniline in Betracht, die zu den korrespondierenden Cyclohexylaminen und Dicyclohexylaminen umgesetzt werden:

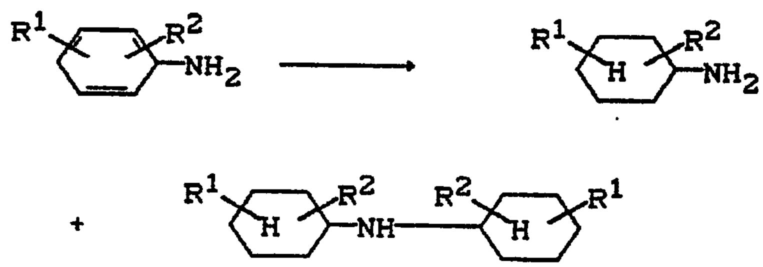

Die Reste $R^1$ und $R^2$ haben unabhängig voneinander die Bedeutung von Wasserstoff, $C_1$-$C_4$-Alkyl oder $C_1$-$C_4$-Alkoxy. Beispiele für die genannten Alkyl- bzw. Alkoxy-substituenten sind: Methyl, Ethyl, Propyl, Isopropyl, Butyl, Isobutyl, Methoxy, Ethoxy, Propoxy, Isopropoxy, Butoxy oder Isobutoxy. In bevorzugter Weise haben die genannten Substituenten 1-2 C-Atome, besonders bevorzugt handelt es sich um Methyl bzw. Methoxy. In weiterhin bevorzugter Weise hat einer der Substituenten $R^1$ und $R^2$ die Bedeutung Wasserstoff, während der andere Substituent Alkyl bzw. Alkoxy im genannten Umfang bedeutet. In besonders bevorzugter Weise richtet sich das Verfahren auf die Kernhydrierung von nichtsubstituiertem Anilin.

Cyclohexylamine und Dicyclohexylamine des genannten Bedeutungsumfanges finden Verwendung zur Herstellung von Alterungsschutzmitteln für Kautschuke und Kunststoffe, als Korrosionsschutzmittel, sowie als Vorprodukte für Pflanzenschutzmittel und Textilhilfsmittel.

Beispiel 1

(Herstellung des Katalysatorträgers nach EP 208 933)

100 g kugelförmiges $\gamma$-$Al_2O_3$ mit einem Durchmesser von 2-5 mm und einer spezifischen Oberfläche von 350 $m^2/g$ wurden in einem Rundkolben vorgelegt und mit einer Lösung von 8,3 g $MnSO_4 \cdot 4H_2O$, 6,2 g $(NH_4)_2Cr_2O_7$ und 45 g Harnstoff in 72 ml Wasser versetzt. Unter drehender Bewegung wurde der Kolben 1 Stunde lang auf 85°C gehalten, die nicht aufgenommene Flüssigkeit abfiltriert, der Katalysatorträger sulfatfrei gewaschen und dann innerhalb von 25 Stunden bei 110°C unter Wasserstrahlvakuum getrocknet. Anschließend wurde der so behandelte Katalysatorträger 30 Minuten bei 300°C getempert.

Beispiel 2

200 g eines nach Beispiel 1 mit Cr und Mn beschichteten $\gamma$-Aluminiumoxids wurden mit einer Lösung getränkt, die aus 2,05 g $RuCl_3$ und 1,67 g $PdCl_2$ in 66 g $H_2O$ hergestellt wurde. Der imprägnierte Katalysator wurde bei 120°C getrocknet und anschließend 2 Stunden bei 250°C im Wasserstoffstrom aktiviert.

25 ml (20,5 g) des so hergestellten Katalysators wurden zur Anilinhydrierung in einem 250 ml-Schüttelautoklaven verwendet, der im Inneren mit einem zentral gelagerten, fest mit dem Autoklaven verbundenen Siebkorb ausgestattet war, in den der Katalysator gefüllt wurde. Mit dieser Katalysatorfüllung wurden je 50 g Anilin unter einem Wasserstoffdruck von 280 bar bei verschiedenen Temperaturen hydriert. Die Hydrierzeit betrug bei allen Versuchen dieser Serie 3 Stunden. Die Hydrierprodukte wurden gaschromatographisch analysiert und zeigten in Abhängigkeit von der Hydriertemperatur folgende Zusammensetzung:

| Temperatur (°C) | 200 | 180 | 160 | 110 |
|---|---|---|---|---|
| Dicyclohexylamin (%) | 54,9 | 23,0 | 12,3 | 6,4 |
| Nebenprodukte (%) | 0,27 | 0,2 | 0,1 | 0 |
| Cyclohexylamin (%) | Rest | Rest | Rest | Rest |

Beispiel 3

300 g eines nach Beispiel 1 mit Cr und Mn beschichteten $\gamma$-$Al_2O_3$ wurden zusätzlich 120 Stunden bei 430°C getempert. 80 g von diesem getemperten Katalysatorträger wurden mit einer Lösung getränkt, die 1,64 g $RuCl_3$ und 1,34 g $PdCl_2$ in 22,4 g Wasser hergestellt wurde. Der imprägnierte Katalysatorträger wurde 2 Stunden bei 250°C in einem Wasserstoffstrom aktiviert und anschließend in fließendem Wasser chlorid-frei gewaschen und erneut bei 120°C getrocknet.

20,5 g (25 ml) des so hergestellten Katalysators wurden in der in Beispiel 2 beschriebenen Weise zur Hydrierung von Anilin in einem 250 ml-Autoklaven eingesetzt. Die ebenfalls bei 280 bar durchgeführten Hydrierungen erbrachten in Abhängigkeit von der Hydriertemperatur folgende Ergebnisse:

| Temperatur (°C) | 200 | 160 | 110 |
|---|---|---|---|
| Dicyclohexylamin (%) | 35,8 | 9,4 | 4,5 |
| Nebenprodukte (%) | 0,17 | 0,14 | < 0,1 |
| Cyclohexylamin (%) | Rest | Rest | Rest |

Beispiel 4

100 g des nach Beispiel 1 mit Cr und Mn beaufschlagten $\gamma$-$Al_2O_3$ wurden mit einer Lösung von 1,03 g $RuCl_3$ und 0,83 g $PdCl_2$ in 34,5 g Wasser getränkt und anschließend bei 120°C getrocknet. Dann wurde der Katalysator 2 Stunden bei 250°C im Wasserstoffstrom aktiviert.

Von diesem so hergestellten Katalysator wurden 25 ml (21 g) für die Hydrierung von Anilin im Autoklaven bei 280 bar eingesetzt. Der Versuchsablauf erfolgte wie in Beispiel 2. Die Hydrierzeit betrug bei jedem dieser Versuche 3 Stunden. In Abhängigkeit von der Hydriertemperatur ergab sich folgende Produktuzusammensetzung:

| Temperatur (°C) | 200 | 160 | 110 |
|---|---|---|---|
| Dicyclohexylamin (%) | 38,9 | 10,6 | 6,9 |
| Nebenprodukte (%) | 0,2 | 0,1 | 0,1 |
| Cyclohexylamin (%) | Rest | Rest | Rest |

Beispiel 5

In weiteren Hydrierversuchen wurden 60 ml (51,3 g) des in Beispiel 3 hergestellten Katalysators in ein senk recht angeordnetes Druckrohr (Durchmesser 14 mm, Länge 70 cm) gebracht, das mit einem Ölthermostat beheizt wurde. Das Zwischenkornvolumen wurde mit feinem Seesand (0,2 bis 0,3 mm) ausgefüllt. Bei 280 bar wurden Anilin und Wasserstoff von oben auf den Katalysator geleitet. Die Flüssigkeit rieselte über den Katalysator nach unten in einen Abscheider. Am Kopf des Abscheiders wurden stündlich 20 l Wasserstoff entspannt. Der Anilindurchsatz entsprach einer Katalysatorbelastung von 0,25 g Anilin/ml Kat. x h und wurde konstant gehalten.

Das Hydrierprodukt wurde in regelmäßigen Zeitabständen aus dem Abscheider entnommen und analysiert. Dabei ergab sich folgende Produktzusammensetzung in Abhängigkeit von der Laufzeit und der Reaktionstemperatur bei Versuchsdauer von mehr als 9 Monaten:

| Laufzeit Stunden | Temperatur °C | Dicyclohexylamin % | Cyclohexylamin % | Nebenprodukte % |
|---|---|---|---|---|
| 92 | 190 | 40,1 | 59,5 | 0,4 |
| 764 | 180 | 31,2 | 68,6 | 0,2 |
| 1.511 | 110 | 9,6 | 90,3 | 0,1 |
| 1.941 | 189 | 42,6 | 57,1 | 0,3 |
| 3.033 | 109 | 8,8 | 91,1 | 0,1 |
| 5.881 | 131 | 15,0 | 84,9 | 0,1 |
| 6.555 | 155 | 23,9 | 75,9 | 0,2 |

Beispiel 6

100 g eines nach Beispiel 1 mit Cr und Mn beschichteten $\gamma$-$Al_2O_3$ wurden mit einer Lösung getränkt, die aus 2,51 g $Ru(NO_3)_3$, 0,33 g $PdCl_2$ und 41 g $H_2O$ hergestellt wurde. Anschließende wurde der Katalysator 2 Stunden bei 250°C im Wasserstoffstrom aktiviert.

60 ml (50,7 g) des so hergestellten Katalysators wurden für die kontinuierliche Anilinhydrierung in ein Druckrohr gefüllt und nach der in Beispiel 5 beschriebenen Weise verfahren. Der Wasserstoffdruck betrug über die gesamte Versuchsdauer 280 bar. Die Hydriertemperatur wurde zwischen 110 und 190°C variiert, um dadurch den Anteil des Dicyclohexylamins im Reaktionsprodukt zu steuern. Die Katalysatorbelastung betrug 0,25 g Anilin/ml • h. Das eingesetzte Anilin wurde vollständig umgesetzt. Das im Verlauf von etwa 270 Tagen ermittelte Hydrierergebnis ist in Fig. 1 graphisch ausgewertet worden. Aufgetragen wurde der vorgegebene Temperaturverlauf sowie der jeweils ermittelte Gehalt an Dicyclohexylamin in Abhängigkeit von den Betriebsstunden des Katalysators. Der Rest (Differenz zu 100 %) bestand im wesentlichen aus Cyclohexylamin.

(zu Beispiel 6): Rieselphasen-Hydrierung von Anilin (Katalysator 0,8%Ru+0,2%Pd auf CrMn-$Al_2O_3$); dargestellt ist die Gewichtsmenge an Dicyclohexylamin in % im Reaktionsgemisch in Abhängigkeit von wechselnden Temperaturen während einer langen Betriebsdauer.

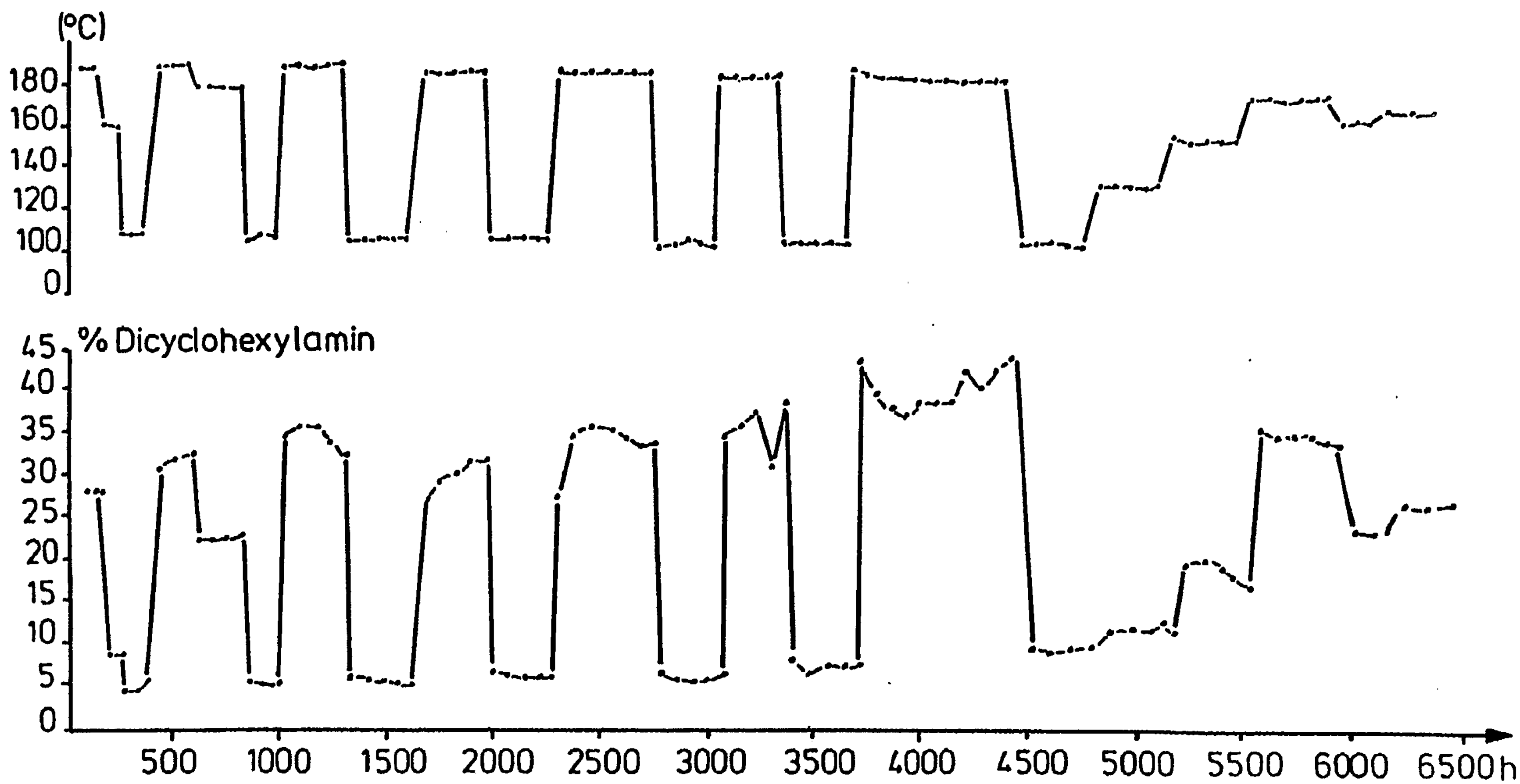

Beispiel 7

200 g eines nach Beispiel 1 mit Cr und Mn beschichteten γ-$Al_2O_3$ (2 bis 5 mm Kugeln) wurden mit einer Lösung getränkt, die aus 2,05 g $RuCl_3$ und 3,33 g $H_2PtCl_6$ in 58 g Wasser hergestellt wurde. Der so imprägnierte Katalysatorträger wurde bei 120°C getrocknet und anschließend 2 Stunden bei 250°C in einem Wasserstoffstrom aktiviert.

25 ml (21 g) des so hergestellten Katalysators wurden in einen 250 ml-Schüttelautoklaven eingebracht und wie in Beispiel 2 beschrieben zur Druckhydrierung (280 bar) von Anilin bei verschiedenen Temperaturen benutzt. Dabei ergab sich in Abhängigkeit von der Temperatur bei einer konstanten Hydrierzeit von 3 Stunden folgende Produktzusammensetzung:

| Temperatur (°C) | 200 | 180 | 160 | 110 |
|---|---|---|---|---|
| Dicyclohexylamin (%) | 46,5 | 21,2 | 13,3 | 9,8 |
| Nebenprodukte (%) | 0,50 | 0,30 | 0,2 | 0,1 |
| Cyclohexylamin (%) | Rest | Rest | Rest | Rest |

**Patentansprüche**

1. Ruthenium-Katalysator, der zusätzlich zum Ruthenium noch Palladium, Platin oder Palladium und Platin enthält, auf einem mit Chrom und Mangan behandelten Träger aus der Gruppe von $Al_2O_3$ und Aluminiumspinell, der die Edelmetalle in einer Gesamtmenge von 0,05-5 Gew.-% und einem Gewichtsverhältnis Ru:Pd, Ru:Pt, beziehungsweise Ru:Pd/Pt von 1:9 – 9:1 enthält, wobei die Prozentsätze auf das Gesamtgewicht des Katalysators bezogen sind.

2. Katalysator nach Anspruch 1, dadurch gekennzeichnet, daß der Edelmetallgehalt 0,05-3 GEw.-%, beträgt.

3. Katalysator nach Anspruch 2, dadurch gekennzeichnet, daß der Edelmetallgehalt 0,1 – 2 Gew.-% beträgt.

4. Katalysator nach Anspruch 1, dadurch gekennzeichnet, daß bis zu 20 Gew.-% der Gesamtmenge an Ruthenium und Palladium durch andere Edelmetalle aus der Gruppe Iridium, Rhodium, Silber und Gold ersetzt sind.

5. Katalysator nach Anspruch 1, dadurch gekennzeichnet, daß die Behandlung des Trägers mit Chrom und Mangan bis zu einem Gehalt von zusammen 0,05-8 Gew.-%, bevorzugt 0,2-5 Gew.-%, bezogen auf das Gesamtgewicht des Katalysators geführt wird und das Gewichtsverhältnis der Elemente Chrom und Mangan auf 5:1 – 1:5, bevorzugt 10:9 – 1:2 eingestellt wird.

6. Verfahren zur Herstellung eines Katalysators nach den Ansprüchen 1 – 5, dadurch gekennzeichnet, daß man auf ein $Al_2O_3$ oder Aluminiumspinell Verbindungen des Chroms und Mangans aufbringt, den so beaufschlagten Träger auf 200 – 450°C erhitzt, dann die Edelmetalle und gegebenenfalls eine basische Alkalimetallverbindung aufträgt und nach jedem Auftragen bei 90 – 130°C trocknet.

7. Verfahren zur Herstellung eines Gemisches von gegebenenfalls substituiertem Cyclohexylamin und gegebenenfalls substituiertem Dicyclohexylamin durch Hydrierung von gegebenenfalls substituiertem Anilin mit Wasserstoff in Gegenwart eines Ruthenium Katalysators, dadurch gekennzeichnet, daß ein Ruthenium-Katalysator, der zusätzlich zum Ruthenium noch Palladium, Platin oder Palladium und Platin enthält, auf einem mit Chrom und Mangan behandelten Träger aus der Gruppe von $Al_2O_3$ und Aluminiumspinell, der die Edelmetalle in einer Gesamtmenge von 0,05 – 5 Gew.-% und einem Gewichtsverhältnis Ru:Pd, Ru:Pt, beziehungsweise Ru:Pd/Pt von 1:9 – 9:1 enthält, wobei die Prozentsätze auf das Gesamtgewicht des Katalysators bezogen sind, eingesetzt und bei 80 – 240°C unter unter höherem Druck gearbeitet wird.

8. Verfahren nach Anspruch 7, dadurch gekennzeichnet, daß man zur Erhöhung des Anteils an Dicyclohexylamin eine höhere Temperatur innerhalb des Bereiches von 80 – 240°C wählt.

9. Verfahren nach Anspruch 7, dadurch gekennzeichnet, daß man eine Katalysatorbelastung von 0,05 – 2 kg, bevorzugt 0,1 – 0,5 kg Anilin pro Liter Katalysator und Stunde einstellt.

10. Verfahren nach Anspruch 7, dadurch gekennzeichnet, daß man ein Anilin der Formel

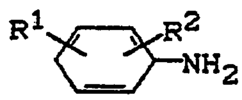

einsetzt, in der
$R^1$ und $R^2$ unabhängig voneinander Wasserstoff, $C_1$-$C_4$-Alkyl oder $C_1$-$C_4$-Alkoxy bedeuten.

**Revendications**

1. Catalyseur au ruthénium, qui contient, outre le ruthénium, du palladium, du platine, ou du palladium et du platine, sur un support traité par le chrome et le manganèse, choisi dans le groupe d'$A1_2O_3$ et du spinelle d'aluminium, qui contient les métaux nobles en quantité totale de 0,05-5% en poids et avec un rapport pondéral Ru:Pd, Ru:Pt, ou Ru:PdlPt dans la plage allant de 1: 9 à 9:1, les pourcentages étant rapportés au poids total de catalyseur.
2. Catalyseur selon la revendication 1, caractérisé en ce que la teneur en métaux nobles s'élève à 0, 05-3% en poids.
3. Catalyseur selon la revendication 2, caractérisé en ce que la teneur en métaux nobles s'élève à 0,1-2% en poids.
4. Catalyseur selon la revendication 1, caractérisé en ce que jusqu'à 20% en poids de la quantité totale de ruthénium et de palladium sont remplacés par d'autres métaux nobles choisis dans le groupe de l'iridium, du rhodium, de l'argent et de l'or.
5. Catalyseur selon la revendication 1, caractérisé en ce que le traitement du support par le chrome et le manganèse est effectué pour obtenir une teneur totale de 0,05 à 8% en poids et, de préférence, de 0,2 à 5% en poids, rapporté au poids total de catalyseur, et que le rapport pondéral des éléments chrome et manganèse est établi dans l'intervalle allant de 5:1 à 1:5 et, de préférence, de 10:9 à 1:2.
6. Procédé de production d'un catalyseur, selon les revendications 1 à 5, caractérisé en ce que l'on dépose sur un $Al_2O_3$ ou un spinelle d'aluminium, des composés du chrome et du manganèse, que le support ainsi traité est chauffé à 200–450°C, puis que les métaux nobles et, éventuellement, un composé basique de métal alcalin, sont déposés et qu'un séchage à 90–130°C est effectué après chaque dépôt.
7. Procédé de production d'un mélange de cyclohexylamine éventuellement substituée et de dicyclohexylamine éventuellement substituée, par hydrogénation d'aniline éventuellement substituée, par l'hydrogène, en présence d'un catalyseur au ruthénium, caractérisé en ce qu'un catalyseur au ruthénium qui contient, outre le ruthénium, du palladium, du platine ou du palladium et du platine, est déposé sur un support traité par le chrome et le manganèse, choisi dans le groupe de $Al_2O_3$ et du spinelle d'aluminium, la teneur totale en métaux nobles étant dans l'intervalle allant de 0,05 à 5% en poids et le rapport pondéral Ru:Pd, Ru:Pt ou Ru:Pd/Pt étant compris dans l'intervalle allant de 1:9 à 9:1, les pourcentages se rapportant au poids total du catalyseur, est utilisé et en opérant sous pression élevée et à 80 – 240°C.
8. Procédé selon la revendication 7, caractérisé en ce que l'on choisit, pour augmenter la proportion de dicyclohexylamine une température plus élevée comprise dans le domaine de 80 à 240°C.
9. Procédé selon la revendication 7, caractérisé en ce que l'on choisit une charge de catalyseur de 0,05 à 2 kg et, de préférence, de 0, 1 à 0, 5 kg d'aniline par litre de catalyseur et par heure.
10. Procédé selon la revendication 7, caractérisé en ce que l'on utilise une aniline suivant la formule

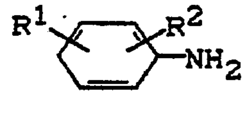

dans laquelle $R^1$ et $R^2$ représente, indépendamment l'un de l'autre, de l'hydrogène, un radical alkyle en $C_1$-$C_4$ ou un radical alkoxy en $C_1$-$C_4$.

**Claims**

1. Ruthenium catalyst also containing palladium, platinum or palladium and platinum in addition to ruthenium on a support treated with chromium and manganese from the group consisting of $Al_2O_3$ and aluminium spinel containing the noble metals in a total amount of 0.05-5% by weight and a weight ratio of Ru:Pd, Ru:Pt, or Ru:Pd/Pt of 1:9 – 9:1, the percentages being based on the total weight of the catalyst.
2. Catalyst according to Claim 1, characterized in that the noble metal content is 0.05-3% by weight.
3. Catalyst according to Claim 2, characterized in that the noble metal content is 0.1 - 2% by weight.
4. Catalyst according to Claim 1, characterized in that up to 20% by weight of the total amount of ruthenium and palladium is replaced by other noble metals from the group consisting of iridium, rhodium, silver and gold.
5. Catalyst according to Claim 1, characterized in that the treatment of the support with chromium and

manganese is carried out up to a combined percentage of 0.05-8% by weight, preferably 0.2-5% by weight, based on the total weight of the catalyst and the weight ratio of the elements chromium and manganese is set to 5:1 – 1:5, preferably 10:9 – 1:2.

6. Process for the preparation of a catalyst according to Claims 1 – 5, characterized in that compounds of chromium and manganese are applied to an $Al_2O_3$ or an aluminium spinel, the support thus treated is heated to 200 – 450°C, the noble metals and, if appropriate, a basic alkali metal compound are then applied and the support is dried at 90 – 130°C after each application.

7. Process for the preparation of a mixture of substituted or unsubstituted cyclohexylamine and substituted or unsubstituted dicyclohexylamine by hydrogenation of substituted or unsubstituted aniline with hydrogen in the presence of a ruthenium catalyst, characterized in that a ruthenium catalyst also containing palladium, platinum or palladium and platinum in addition to ruthenium on a support treated with chromium and manganese from the group consisting of $A1_2O_3$ and aluminium spinel containing the noble metals in a total amount of 0.05-5% by weight and a weight ratio of Ru:Pd, Ru:Pt, or Ru:Pd/Pt of 1:9 – 9:1, the percentages being based on the total weight of the catalyst, is used and the reaction is carried out at 80 – 240°C at an elevated pressure.

8. Process according to Claim 7, characterized in that a higher temperature within the range of 80 – 240°C is chosen to increase the dicyclohexylamine percentage.

9. Process according to Claim 7, characterized in that the space velocity of the catalyst is set to 0.052 kg, preferably 0.1 – 0.5 kg, of aniline per litre of catalyst per hour.

10. Process according to Claim 7, characterized in that an aniline of the formula

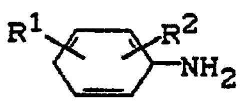

is used in which
$R^1$ and $R^2$ independently of one another denote hydrogen, $C_1$-$C_4$-alkyl or $C_1$-$C_4$-alkoxy.